# EUROPEAN PATENT APPLICATION

(11) **EP 4 613 737 A1**
(43) Date of publication of application: **10.09.2025**
(21) Application number: 24161644.0
(22) Date of filing: 06.03.2024
(51) Int. Cl.: C07C 235/82, C08G 71/00

(54) **DENTAL COMPOSITION**

(71) Applicant: DENTSPLY SIRONA Inc., York, PA 17401 (US); DENTSPLY DETREY GmbH, 78467 Konstanz (DE)
(72) Inventor: Trötschler, Tobias, 78467 Konstanz (DE); Fredrich, Sebastian, 78467 Konstanz (DE)
(74) Representative: Crow, Martin

(57) **Abstract**

A polymerizable composition comprising a mixture of
(a) at least one polymerizable monomer having a polymerizable carbon-carbon double bond;
(b) an initiator system comprising a compound of the following formula (I), or a salt thereof:

CQ-Ar (I)

wherein CQ is a group of the following formula (II) 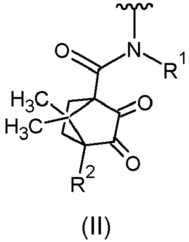 and Ar is a group of the following formula (III): 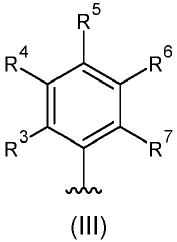 wherein
R¹
represents a hydrogen atom, or a C₁₋₆ alkyl group;
R²
represents a hydrogen atom or a group CONR⁸-Ar', wherein R⁸ is a hydrogen atom, or a C₁₋₆ alkyl group, and Ar' is independently a group of formula (III);
R³, R⁴, R⁵, R⁶, and R⁷
which may be the same or different, are independently selected from a hydrogen atom, a C₁₋₈ alkyl group, a nitro group, a cyano group, an amino group optionally substituted by one or two C₁₋₆ alkyl groups which may be the same or different, a group COOR⁹, wherein R⁹ is a hydrogen atom, or a C₁₋₆ alkyl group, and a group of formula (II), wherein R¹ independently is as defined for formula (II) and R² is a hydrogen atom; with the proviso that not more than two of R³, R⁴, R⁵, R⁶, and R⁷ may be a group of formula (II) and/or a group COOR⁹.

## Description

### Field of the Invention

The present invention relates to a polymerizable composition comprising a specific polymerization initiator system containing a compound being a combination of a photoinitiator and a specific aromatic amine compound. The present invention also relates to the specific polymerization initiator compound. Furthermore, the present invention also relates to the use of the polymerization initiator compound in a photocurable dental composition. The compound of the present invention is highly active as an initiator compound in a polymerization reaction of a monomer having a polymerizable carbon-carbon double bond while at the same time having a reduced toxicity. The polymerizable composition may advantageously be a dental composition having a reduced risk for adverse effects on the patient.

### Background of the Invention

Polymerizable compositions are known. Generic compositions contain polymerizable monomers, an initiator system and optionally further components depending on the particular use. In dental applications, the polymerizable composition may further contain particulate fillers or solvents as further components.

Initiator systems may be activated chemically, thermally, or by irradiation. When the initiator system is adapted to be activated by irradiation only, the initiator system may be stored in admixture with the polymerizable monomers so that mixing of multiple components prior to use is avoided. During photocuring of a composition containing polymerizable monomers, the photoinitiator system generates free radicals upon exposure to visible light. Free radicals may be typically produced by either of two pathways:
(1) the photoinitiator compound undergoes excitation by energy absorption with subsequent decomposition of the compound into one or more radicals (Norrish type I), or
(2) the photoinitiator compound undergoes excitation and the excited photoinitiator compound interacts with a second compound by either energy transfer or a redox reaction to form free radicals from any of the compounds (Norrish type II).

To be useful in a clinical setting, the photocuring of a generic polymerizable dental composition must meet in combination a number of conditions:
- The rate of polymerization must be sufficiently high so that polymerization may be accomplished within about 30-40 seconds to avoid discomfort on the part of the patient.
- The spectrum and intensity of light source required for irradiation must not harm any hard or soft oral tissues of the patient.
- The heat of polymerization must be low to avoid damage of the pulp tissue via heat generated from absorption and conversion into thermal energy.
- Polymerization of the bulk of the restoration and the interface layer to the residual dental tissue must be complete since unreacted monomers can have an adverse effect on soft dental tissue.
- The components of the polymerizable dental composition should not cause any toxic, inflammatory, allergic, carcinogenic or mutagenic reactions in the patient.

In addition, photoinitiator systems often are required to have a high acid resistance, solubility, thermal stability, and storage stability when incorporated into a dental composition.

Finally, given that dental compositions usually contain (meth)acrylate or (meth)acrylamide monomers, free radical photocuring may be inhibited by the presence of oxygen. Oxygen inhibition is due to the rapid reaction of propagating radicals with oxygen molecules to yield peroxyl radicals which are not as reactive towards carbon-carbon unsaturated double bonds and therefore do not initiate or participate in any photopolymerization reaction. Oxygen inhibition may lead to premature chain termination and, therefore, incomplete photocuring. Nevertheless, a certain degree of oxygen inhibition on the top surface of the adhesive layer is required for the bonding to the adjacent restorative.

Despite great efforts of researchers in the field, the number of effective and safe photoinitiator systems for polymerizable dental compositions is limited. In fact, a great number of dental compositions rely on a combination of camphorquinone (CQ) and aromatic amines such as N,N-dimethyl-p-toluidine or ethyl 4-(dimethylamino)benzoate. CQ as such can photoinitiate polymerization only at a low rate whereby the aromatic amine is required as a coinitiator for accelerating the polymerization. It is assumed that the formation of aminoalkyl radicals occurs via photoinduced electron transfer whereby the interaction of CQ with the coinitiator forms an aminoalkyl radical which initiates the polymerization of the polymerizable monomers. For this purpose, the reactive aminoalkyl radical must approach the unsaturated bond in the monomer.

Although aromatic amines such as N,N-dimethyl-p-toluidine or ethyl 4-(dimethylamino)benzoate are advantageous in promoting polymerization, the compound gives rise to concerns regarding the safety of the patient.

It is the problem of the present invention to provide a polymerizable composition wherein the components, notably the initiator does not give rise to concerns regarding the safety of the user, notably a patient, while at the same time the initiator system provides a rate of polymerization so that polymerization may be accomplished in a reasonably short time, the spectrum and intensity of light source required for irradiation does not harm any hard or soft oral tissues of the patient, the heat of polymerization is low to avoid damage of the pulp tissue via heat conversion in thin layers, and polymerization of the inner part of the restoration is complete and unreacted monomers are avoided.

### Summary of the Invention

The present invention provides a polymerizable composition comprising a mixture of
(a) at least one polymerizable monomer having a polymerizable carbon-carbon double bond;
(b) an initiator system comprising a compound of the following formula (I), or a salt thereof:

   CQ-Ar (I)

   wherein CQ is a group of the following formula (II) 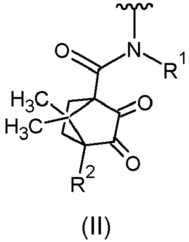
   and Ar is a group of the following formula (III): 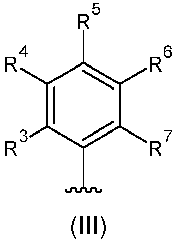
   wherein
      - R¹: represents a hydrogen atom, or a C₁₋₆ alkyl group;
      - R²: represents a hydrogen atom or a group CONR⁸-Ar', wherein R⁸ is a hydrogen atom, or a C₁₋₆ alkyl group, and Ar' is independently a group of formula (III);
      - R³, R⁴, R⁵, R⁶, and R⁷: which may be the same or different, are independently selected from a hydrogen atom, a C₁₋₈ alkyl group, a nitro group, a cyano group, an amino group optionally substituted by one or two C₁₋₆ alkyl groups which may be the same or different, a group COOR⁹, wherein R⁹ is a hydrogen atom, or a C₁₋₆ alkyl group, and a group of formula (II), wherein R¹ independently is as defined for formula (II) and R² is a hydrogen atom; with the proviso that not more than two of R³, R⁴, R⁵, R⁶, and R⁷ may be a group of formula (II) and/or a group COOR⁹.

The present invention is based on the recognition that a compound of formula (I) provides a rate of polymerization which is sufficiently high so that polymerization of a polymerizable dental composition may be accomplished within at most 30-40 seconds while the irradiation spectrum and intensity of a conventional light source may be used which is considered to be safe for hard or soft oral tissues. Moreover, the heat of polymerization may be controlled by using a compound of formula (I) to avoid damage of the pulp tissue via heat conversion in thin layers. Furthermore, the use of a compound of formula (I) provides high conversion so that the polymerization of the inner part of the restoration is essentially complete. Finally, a compound of formula (I) is not expected to cause any toxic, inflammatory, allergic, carcinogenic or mutagenic reactions in the patient.

The present invention also provides a compound of the following formula (I), or a salt thereof:

CQ-Ar (I)

wherein CQ and Ar are as defined above. Finally, the present invention provides a use of the compound of the formula (I), or a salt thereof in a photocurable dental composition.

A polymerizable dental composition according to the present invention contains a compound of formula (I) as an initiator which is highly effective, and does not result in toxic, inflammatory, allergic, carcinogenic or mutagenic reactions in a patient.

### Brief Description of the Figures

Fig. 1 shows the mechanical properties of a dental composition containing an initiator system of the present invention compared to the mechanical properties of a dental composition containing camphorquinone.
Fig. 2 is an ¹H-NMR spectrum of N,7,7-Trimethyl-2,3-dioxo-N-phenylbicyclo[2.2.1.]heptan-1-carboxamide.

### Detailed Description of the Preferred Embodiments

The terms "polymerization" or "polymerizable" relate to the combining by covalent bonding of many smaller molecules, such as monomers in the form of radical-polymerizable or cationically polymerizable compounds, to form larger molecules, that is, macromolecules or polymers. The monomers may be combined to form only linear macromolecules or they may be combined to form three-dimensional macromolecule, commonly referred to as crosslinked polymers. In case of a higher conversion rate of the polymerizable monomer, the amount of multifunctional monomers may be reduced or the leaching problem may be alleviated.

The term "photocurable" refers to a dental composition that will radically and/or cationically polymerize into a crosslinked polymer network when irradiated for example with actinic radiation such as ultraviolet (UV), visible, or infrared radiation. "Actinic radiation" is any electromagnetic radiation that is capable of producing photochemical action and can have a wavelength of at least 150 nm and up to and including 1250 nm, and typically at least 300 nm and up to and including 750 nm.

The term "radical-polymerizable" as used herein in connection with compounds (a) means any compound capable of radical polymerization. Typically, compounds (a) are radical-polymerizable due to a polymerizable double bond, preferably one or more carbon-carbon double bonds. Examples of the polymerizable double bond include vinyl, conjugated vinyl, allyl, acryl, methacryl and styryl. More preferably, the polymerizable double bound is selected from the group consisting of acryl, methacryl and styryl. Acryl and methacryl may be (meth)acryloyl or (meth)acrylamide. Most preferably, for compounds (a), the polymerizable double bound is acryl or methacryl.

The term "cationically polymerizable" as used herein in connection with compounds (a) means any compound capable of cationic polymerization. Typically, compounds (a) are cationically polymerizable due to the presence of a functional group having a carbon-carbon double bond such as a vinyl ether group.

The term "polymerization initiator system" refers to a system comprising (i) a photoinitiator compound and (ii) a coinitiator compound. Optionally, the polymerization initiator system may further comprise (iii) an additional electron-donor.

The term "photoinitiator" used in connection with the compound (i) refers to a compound that is activated, for example by forming free radicals, typically by exposure to light or interaction with another compound such as coinitiator compound (ii) and/or electron-donor (iii) in a photochemical process.

The present invention relates to a polymerizable, notably a photocurable dental composition. The photocurable dental composition may be a dental composite, a dental adhesive, a dental glass ionomer cement, or a dental impression material.

### The polymerizable compounds (a)

The photocurable dental composition comprises (a) one or more polymerizable compounds. The compounds may be radical-polymerizable or cationically polymerizable.

The one or more radical-polymerizable compounds (a) may preferably be radical-polymerizable N-substituted alkyl acrylic or acrylic acid amide monomers or a (meth)acrylate compounds.

A radical-polymerizable N-substituted alkyl acrylic or acrylic acid amide monomer may be preferably selected from compounds characterized by one of the following formulae (X), (XI) and (XII):

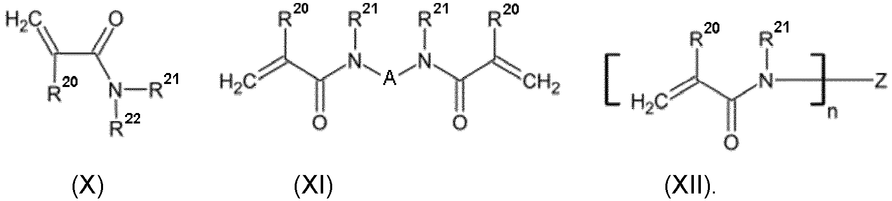

In formulae (X), (XI) and (XII), R²⁰, R²¹ and R²² independently represent a hydrogen atom or a C1 to C8 alkyl group; A represents a divalent substituted or unsubstituted organic residue having from 1 to 10 carbon atoms, whereby said organic residue may contain from 1 to 3 oxygen and/or nitrogen atoms; Z represents a saturated at least trivalent substituted or unsubstituted C1 to C8 hydrocarbon group, a saturated at least trivalent substituted or unsubstituted cyclic C3 to C8 hydrocarbon group, and n is at least 3.

Preferably, the one or more radical-polymerizable compounds (a) include bisacrylamides such as N,N'-diethyl-1,3-bisacrylamido-propan (BADEP), 1,3-bisacrylamido-propan (BAP), 1,3-bisacrylamido-2-ethyl-propan (BAPEN), N,N'-(2E)-2-butene-1,4-diylbis[N-2-propen-1-yl-2-propenamide] (BAABE), N,N-di(cyclopropyl acrylamido) propane (BCPBAP) and N,N'-(2-hydroxy-1,3-propanediyl)bis[*N*-2-propen-1-yl-2-propenamide] (DAAHP).

Alternatively or additionally, for the one or more radical-polymerizable compounds (a), a (meth)acrylate compound may be selected from the group of methyl acrylate, methyl methacrylate, ethyl acrylate, ethyl methacrylate, propyl acrylate, propyl methacrylate, isopropyl acrylate, isopropyl methacrylate, 2-hydroxyethyl acrylate, 2-hydroxyethyl methacrylate (HEMA), hydroxypropyl acrylate, hydroxypropyl methacrylate, tetrahydrofurfuryl acrylate, tetrahydrofurfuryl methacrylate, glycidyl acrylate, glycidyl methacrylate, the diglycidyl methacrylate of bis-phenol A ("bis-GMA"), glycerol mono-and di- acrylate, glycerol mono- and dimethacrylate, ethyleneglycol diacrylate, ethyleneglycol dimethacrylate, polyethyleneglycol diacrylate (where the number of repeating ethylene oxide units vary from 2 to 30), polyethyleneglycol dimethacrylate (where the number of repeating ethylene oxide units vary from 2 to 30 especially triethylene glycol dimethacrylate ("TEGDMA"), neopentyl glycol diacrylate, neopentylglycol dimethacrylate, trimethylolpropane triacrylate, trimethylol propane trimethacrylate, mono-, di-, tri-, and tetra- acrylates and methacrylates of pentaerythritol and dipentaerythritol, 1,3-butanediol diacrylate, 1,3-butanediol dimethacrylate, 1,4-butanedioldiacrylate, 1,4-butanediol dimethacrylate, 1,6-hexane diol diacrylate, 1,6-hexanediol dimethacrylate, di-2-methacryloyloxethyl hexamethylene dicarbamate, di-2-methacryloyloxyethyl trimethylhexanethylene dicarbamate, di-2-methacryloyl oxyethyl dimethylbenzene dicarbamate, methylene-bis-2-methacryloxyethyl-4-cyclohexyl carbamate, di-2-methacryloxyethyl-dimethylcyclohexane dicarbamate, methylene-bis-2-methacryloxyethyl-4-cyclohexyl carbamate, di-1-methyl-2-methacryloxyethyl-trimethyl-hexamethylene dicarbamate, di-1-methyl-2-methacryloxyethyl-dimethylbenzene dicarbamate, di-1-methyl-2-methacryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-2-methacryloxyethyl-4-cyclohexyl carbamate, di-1-methyl-2-methacryloxyethyl-hexamethylene dicarbamate, di-1-methyl-2-methacryloxyethyl-trimethylhexamethylene dicarbamate, di-1-methyl-2-methacryloxyethyl-dimethylbenzene dicarbamate, di-1-methyl-2-metha-cryloxyethyl-dimethylcyclohexane dicarbamate, methylene-bis-1-methyl-2-methacryloxyethyl-4-cyclohexyl carbamate, di-1-chloromethyl-2-methacryloxyethyl-hexamethylene dicarbamate, di-1-chloromethyl-2-methacryloxyethyl-trimethylhexamethylene dicarbamate, di-1-chloromethyl-2-methacryloxyethyl-dimethylbenzene dicarbamate, di-1-chloromethyl-2-methacryloxyethyl-dimethylcyclohexane dicarbamate, methylene-bis-1-chloromethyl-2-methacryloxyethyl4-cyclohexyl carbamate, 2,2'-bis(4-methacryloxyphenyl)propane, 2,2'bis(4-acryloxyphenyl)propane, 2,2'-bis[4(2-hydroxy-3-methacryloxy-phenyl)]propane, 2,2'-bis[4(2-hydroxy-3-acryloxy-phenyl)propane, 2,2'-bis(4-methacryloxyethoxyphenyl)propane, 2,2'-bis(4-acryloxyethoxyphenyl)propane, 2,2'-bis(4-methacryloxypropoxyphenyl)propane, 2,2'-bis(4-acryloxypropoxyphenyl)propane, 2,2'-bis(4-methacryloxydiethoxyphenyl)propane, 2,2'-bis(4-acryloxydiethoxyphenyl)propane, 2,2'-bis[3(4-phenoxy)-2-hydroxypropane-1-methacrylate]propane,and 2,2'-bis[3(4-phenoxy)-2-hydroxypropane-1-acryalte]propane, may be mentioned. Other suitable examples of radical-polymerizable components are isopropenyl oxazoline, vinyl azalactone, vinyl pyrrolidone, styrene, divinylbenzene, urethane acrylates or methacrylates, epoxy acrylates or methacrylates and polyol acrylates or methacrylates.

Preferably, the one or more radical-polymerizable compounds (a) contain one or two radical-polymerizable groups, more preferably two radical-polymerizable groups, such as acrylamides or bisacrylamides like Bis-GMA, TEGDMA, BADEP, BAP, BAPEN, BAABE, BCPBAP and DAAHP.

It is preferable that a blending ratio of the one or more radical-polymerizable compounds (a) to the entire photocurable dental composition is 5 to 80% by weight. More preferably, the blending ratio is 10 to 60% by weight.

### The polymerization initiator system

The polymerizable dental composition further comprises a polymerization initiator system which is preferably soluble in the mixture. Specifically, the polymerization initiator may be fully dissolved in the required amount in the photocurable dental composition. Typically, the polymerizable dental composition contains 0.01 to 20 percent by weight, more preferably 0.1 to 10 percent by weight based on the total amount of the composition of the polymerization initiator system.

The polymerization initiator system comprises a photoinitiator compound of formula (I). Preferably, the photoinitiator compound has a light absorption maximum in the range from 300 to 800 nm, in particular 350 to 500 nm. The polymerization initiator system may comprise one or a mixture of two or more photoinitiator compound(s) of the present invention, or photoinitiator compound(s) of the present invention and one or more additional photoinitiator compound(s) such as an acylphosphine oxide or a 1,2-diketone. Suitable 1,2-diketone photoinitiator compounds may be selected from the group consisting of camphorquinone, benzil, 2,2'-3 3'- and 4,4'-dihydroxylbenzil, 2,3-butanedione, 2,3-pentanedione, 2,3-hexanedione, 3,4-hexanedione, 2,3-heptanedione, 3,4-heptanedione, 2,3-octanedione, 4,5-octanedionefuril, 1,2-cyclohexanedione, 1,2-naphthaquinone, and acenaphthaquinone. Camphorquinone is preferred.

Typically, the polymerizable dental composition contains 0.005 to 5 percent by weight, more preferably 0.05 to 3 percent by weight based on the total amount of the composition of the photoinitiator compound of formula (I) according to the present invention.

The compound of the present invention, or a salt thereof contained in the initiator system has the following formula (I):

CQ-Ar (I)

In formula (I), CQ is a group of the following formula (II):

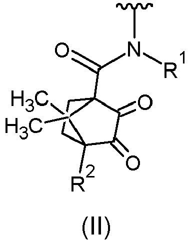

The group of formula (II) is derived from camphor quinone whereby the bridgehead methyl group of camphor quinone is transformed into an amide group linking an aromatic amine coinitiator to the camphor quinone group.

In a compound of formula (I), Ar is a group of the following formula (III): 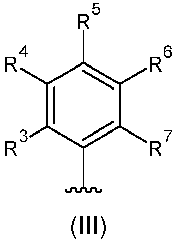 which is an aromatic group combining with a group of formula (II) to an aromatic amide group.

In a compound of formula (I), R¹ represents a hydrogen atom, or a C₁₋₆ alkyl group. According to a preferred embodiment, R¹ represents a C₁₋₆ alkyl group, notably a C₁₋₃ alkyl group. Most preferably, R¹ represents a methyl group.

In a compound of formula (I), R² represents a hydrogen atom or a group CONR⁸-Ar', wherein R⁸ is a hydrogen atom, or a C₁₋₆ alkyl group, and Ar' is independently a group of formula (III). According to a preferred embodiment, R² is a hydrogen atom.

According to a particularly preferred embodiment, R¹ is a methyl group and R² is a hydrogen atom.

When R² represents a group CONR⁸-Ar', then the compound of formula (I) contains a further group derived from an aromatic amine group at a bridgehead position. In the group CONR⁸-Ar', Ar' is a further group of formula (III) wherein the groups R³, R⁴, R⁵, R⁶, and R⁷ may be the same or different from R³, R⁴, R⁵, R⁶, and R⁷ in a group Ar of formula (III). When R² contains a further group derived from an aromatic amine group at a bridgehead position, a compound of formula (I) may support a second polymerization.

In a compound of formula (III), R³, R⁴, R⁵, R⁶, and R⁷ which may be the same or different, are independently selected from a hydrogen atom, a C₁₋₈ alkyl group, a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, or a group of formula (II), wherein R¹ independently is as defined for formula (II) and R² is a hydrogen atom; with the proviso that not more than two of R³, R⁴, R⁵, R⁶, and R⁷ may be a group of formula (II) and/or a group COOR⁹.

According to a preferred embodiment, R³, R⁴, R⁵, R⁶, and R⁷ which may be the same or different, are independently selected from a hydrogen atom and a C₁₋₈ alkyl group. For example, R³, R⁴, R⁵, R⁶, and R⁷ may represent hydrogen atoms.

According to a further preferred embodiment, one of R³, R⁴, R⁵, R⁶, and R⁷ is a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, or a group of formula (II) wherein R¹ independently is as defined for formula (II) and R² is a hydrogen atom, and the remaining of R³, R⁴, R⁵, R⁶, and R⁷ are hydrogen atoms.

Preferably, the compound of formula (I) has the following structure, or a salt thereof:

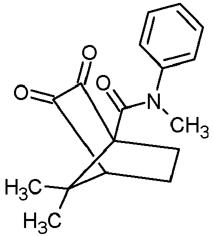

A compound of the present invention may be prepared according to standard synthetic methods. In general, the synthetic procedure involves the reaction of an acyl chloride or its derivative with an aromatic amine in the presence of a base, under an inert atmosphere, followed by purification of the product.

More specifically, an acyl chloride of the following formula (IV) is selected as a starting material: 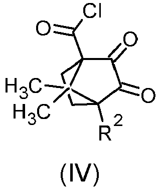 wherein R² is as defined for a compound of formula (II). According to a preferred embodiment, the acyl chloride is 7,7-Dimethyl-2,3-dioxobicyclo[2.2.1]heptane-1-carbonyl chloride.

The acyl chloride is dissolved in a suitable organic solvent. Examples of suitable solvents are dichloromethane, DMSO, tetrahydrofuran, diethyl ether and ethyl acetate. Preferably, the reaction is carried out under an inert gas atmosphere such as nitrogen or argon.

A suitable base, such as pyridine, is then added to the solution to neutralize the HCl formed during the reaction and to facilitate the reaction by acting as a catalyst. The choice of base can vary depending on the specific requirements of the reaction, such as the solubility of the base in the chosen solvent and its reactivity with the specific acyl chloride used.

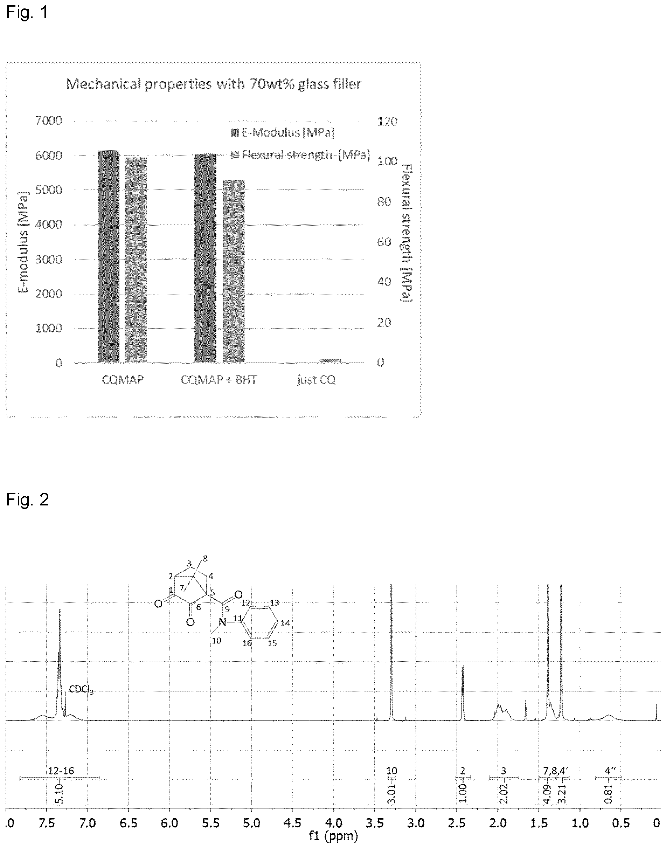

Subsequently, an aromatic amine is added to the reaction mixture. The aromatic amine is a compound of the following formula (V): 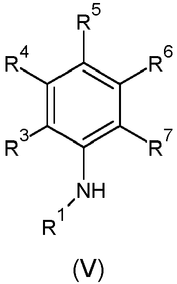 wherein R¹, and R³ to R⁷ are defined for a compound of formula (I). If necessary, functional groups such as an additional amino group or a carboxyl group may be protected during the reaction. The amine of formula (V) reacts with the acyl chloride of formula (IV) to form an amide bond. The reaction conditions are not particularly limited. For example, the reaction mixture may be stirred at a temperature from 0°C to the boiling point of the solvent, preferably at room temperature. The reaction time can vary but is typically around 1 to 72, more preferably about 48 hours to ensure completion of the reaction. Adjustments may be necessary based on the reactivity of the specific reactants. After the reaction is complete, the mixture may be diluted with additional solvent, if necessary, and washed with a saturated solution of a base such as NaHCOs followed by brine to remove impurities. The organic phase may be dried over an anhydrous salt, such as Na₂SO₄, to remove any remaining water. The product may be precipitated from the solution using a suitable solvent mixture, such as n-hexane/ethyl acetate. The ratio of solvents can be adjusted based on the solubility of the product to optimize yield and purity. The desired product may be filtered or centrifuged to isolate the product. The product may be characterized using appropriate analytical techniques, such as NMR, IR, MS, or chromatography, to confirm its structure and purity. Figure 2 shows an ¹H-NMR spectrum of N,7,7-Trimethyl-2,3-dioxo-N-phenylbicyclo[2.2.1.]heptan-1-carboxamide prepared in the examples.

The generalized procedure can be adapted for the synthesis of a wide range of compounds by selecting appropriate reactants, solvents, bases, and amines, and by adjusting the reaction conditions to suit the specific chemical transformations required for the synthesis of the target compound.

Typically, a polymerizable composition contains 0.005 to 10 percent by weight, more preferably 0.05 to 5 percent by weight, still more preferably 0.1 to 3 percent by weight based on the total amount of the composition of the compound of formula (I).

According to a preferred embodiment, a polymerizable dental composition according to the present invention contains the compound of formula (I) in an amount of from 0.05 to 5.00 percent by weight based on the total weight of the polymerizable dental composition.

### Further components

Optionally, the photocurable dental composition of the present invention may further comprise a solvent and/or a particulate filler.

According to a preferred embodiment, the polymerizable composition according to the present invention further comprises a solvent. Suitable solvents may be selected from water, alcohols such as methanol, ethanol, propanol (n-, i-), butanol (n-, iso-, tert-), ketones such as acetone or the like.

The polymerizable dental composition of the present invention may preferably comprise 5 to 75 percent by weight based on the total weight of the composition of a solvent.

According to a preferred embodiment, the polymerizable composition according to the present invention further comprises a particulate filler. Suitable particulate fillers may be selected from fillers currently used in dental compositions. The filler should be finely divided and preferably has a maximum particle diameter less than about 100 µm and an average particle diameter less than about 10 µm. The filler may have a unimodal or polymodal (e.g., bimodal) particle size distribution.

The filler can be an inorganic material. It can also be a crosslinked organic material that is insoluble in the one or more radical-polymerizable compounds (a), and is optionally filled with inorganic filler. The filler can be radiopaque. Examples of suitable particulate inorganic fillers are naturally-occurring or synthetic materials such as quartz, nitrides such as silicon nitride, glasses derived from, for example Ce, Sb, Sn, Zr, Sr, Ba and Al, colloidal silica, feldspar, borosilicate glass, kaolin, talc, titania, and zinc glass, and submicron silica particles such as pyrogenic silicas. Examples of suitable non-reactive organic filler particles include filled or unfilled pulverized polycarbonates or polyepoxides. Preferably the surface of the filler particles is treated with a coupling agent in order to enhance the bond between the filler and the matrix. The use of suitable coupling agents include gamma-methacryloxypropyltrimethoxysilane, gamma-mercaptopropyltriethoxysilane, gamma-aminopropyltrimethoxysilane, and the like.

The particulate filler may also be a hybrid organic/inorganic filler, for example a SphereTEC^{®} filler obtainable by a process for the preparation of composite filler particles, comprising:
a) coating a particulate filler having a median particle size (D50) of from 1 to 1200 nm with a coating composition containing a film-forming agent forming a coating layer on the surface of the particulate filler, said coating layer displaying reactive groups on the surface of the coating layer, said reactive groups being selected from addition polymerizable groups and step-growth polymerizable groups, thereby forming a coated particulate filler; subsequently or concurrently
b) agglomerating the coated particulate filler, optionally in the presence of a further crosslinking agent and optionally in the presence of a further particulate filler not displaying reactive groups, for providing a granulation of the coated particulate filler wherein the granulation contains the coated particulate filler particles and the optional further particulate filler particles separated from and connected to each other by at least one coating layer, whereby the at least one coating layer may be crosslinked by crosslinking groups obtained by reacting the reactive groups and optionally a further crosslinking agent;
c) optionally milling, classifying and/or sieving the granulation of the coated particulate filler; and
d) optionally further crosslinking the granulation of the coated particulate filler; for providing composite filler particles having a median particle size (D50) of from 1 to 70 µm, wherein reactive groups are transformed into crosslinking groups obtained by reacting reactive groups and optionally a further crosslinking agent, and wherein the particulate filler is the main component by volume of the composite filler particles as further described in EP-A 2 604 247.

Furthermore, the filler may be a glass filler. The glass filler is preferably a silanated glass filler. The surface of the glass may be modified by using a surface modifying agent. The surface modifying agent contains a modifying compound capable of reacting with surface atoms of the filler, thereby forming a covalent bond between the surface atoms of the filler and the modifying compound. Additionally, the modifying compound may contain one or more polymerizable double bonds reactive in the crosslinking reaction after the filler is modified. The modifying agent may contain one or more modifying compounds. Preferably, the modifying compound provides a polymerizable ligand capable of crosslinking which may be a compound of one of the following formulae (VI), (VII) and (VIII), or a hydrolysis product thereof

Xᵣ R¹⁰₃₋ᵣSiL (VI)

Xᵣ R¹⁰₂₋ᵣSiL'L" (VII)

XᵣSiL'L"L‴ (VIII)

wherein
X represents a hydrolyzable group;
R¹⁰ represents an alkyl, cycloalkyl, cycloalkylalkyl, aralkyl or aryl group,
L, L', L", and L‴
which may be the same or different represent independent from each other an organic group containing one or more polymerizable double bonds;
r is an integer of 1 to 3,
whereby the sum of X, R¹⁰, L, L', L", and L‴ is 4 for each of formula (VI), (VII), and (VIII).

Preferably, X is a halogen atom or OR¹¹, wherein R¹¹ is an alkyl, cycloalkyl, cycloalkylalkyl, aralkyl or aryl group. More preferably, R¹⁰ or R¹¹ are independently an alkyl group.

In order to impart crosslinking capability to the organofunctional silicon compound, L, L', L", and L‴ contain one or more polymerizable double bonds capable of taking part in a crosslinking reaction. In a preferred embodiment, L, L', L", and L‴ may be selected from the group of allyl, (meth)acrylic ester groups, and (meth)acrylic amide groups.

The polymerizable dental composition of the present invention may preferably comprise 0.1 to 85 percent by weight based on the total weight of the composition of particulate filler.

The polymerizable dental compositions of the present invention may further contain stabilizers, pigments, free radical scavengers, accelerators, polymerization inhibitors, reactive and nonreactive diluents, coupling agents to enhance reactivity of fillers, rheology modifiers, and surfactants.

According to a preferred embodiment, the polymerizable composition according to the present invention further comprises a polymerization accelerator, preferably a diphenyliodonium salt. The polymerizable composition or polymerizable dental composition may contain 0.005 to 5 percent by weight, more preferably 0.05 to 3 percent by weight, still more preferably 0.1 to 2 percent by weight based on the total amount of the composition of an additional accelerator such as a diphenyl iodonium salt (DPI).

Suitable stabilizers may be selected from reducing agents such as vitamin C, inorganic sulfides and polysulfides and the like.

According to a preferred embodiment, the polymerizable composition of the present invention further comprises a polymerization inhibitor. Suitable polymerization inhibitors may be selected from hydroquinone monomethylether, 2,6-di-tert.-butyl-p-cresol, tetramethyl piperidine N-oxyl radical, galvinoxyl radical, or polymerization inhibitors disclosed in EP-A 1776943. The polymerizable dental composition of the present invention may preferably comprise 0.001 to 5 percent by weight more preferably 0.01 to 2 percent by weight based on the total weight of the composition of the polymerization inhibitor.

Suitable pigment may be selected from the group comprising iron oxide, titanium oxide, barium sulfate, manganese oxide, zinc oxide, bismuth oxide and organic fluorophores.

In the preferred embodiment of the present invention, the dental composition comprises 10 to 99.5% by weight of the at least one polymerizable monomer, 0.1- 3% by weight of the at least one polymerization initiator, 0.05 to 5% by weight of the at least one polymerization coinitiator which is a compound of formula (I), 0.001-3.0% by weight of the at least one polymerization inhibitor and 0-85% by weight of the at least one filler.

### One-part or multi-part composition

The polymerizable dental composition according to the present invention may be a one-part or a multi-part polymerizable dental composition.

The term "one-part" as used herein means that all components of the polymerizable dental composition are comprised in one single part.

The term "multi-part" as used herein means that the components of the polymerizable dental composition are comprised in a multitude of separate parts. For example, a first part of components is comprised in a first part, while as second part of components is comprised in a second part, a third part of components may be comprised in a third part, a fourth part of components may be comprised in a fourth part, and so on.

Preferably, the polymerizable dental composition is a one-part or a two-part polymerizable dental composition, most preferably a one-part polymerizable dental composition.

### Use of the present polymerization initiator of formula (I)

The compound of formula (I) or a salt thereof as described above may preferably be used in a photocurable dental composition, preferably a polymerizable dental composition selected from a dental composite, a temporary sealing material, a dental cement, a dental adhesive, and a dental fissure sealant. The compound of formula (I) is preferably part of an initiator system as described herein.

The present invention will now be further explained with the following examples and comparative examples.

### Examples

The following abbreviations and terms are used in the present specification:
UDMA= Urethane dimethacrylate resin
DMABE = N,N-Dimethylamino ethyl benzoate
DPI = Dimethyl diphenyl iodonium hexafluorophosphate
EBPADMA = Ethoxylated bisphenol A dimethacrylate
BAABE = N,N'-diallyl-1,4- bisacrylamido-(2E)-but-2-ene
BADEP = N,N'-diethyl-1,3-bisacrylamido-propane
CQ = Camphorquinone
BHT = Butylated hydroxytoluene
Paste = A dental composition comprising a filler
TEGDMA = Triethylenglycol dimethacrylate
BisGMA = Bisphenol A-glycidyl methacrylate
Isomers in the sense of this invention are stereoisomers, including enantiomers. Inhibitor in the sense of this invention is a polymerization inhibitor.
CQMAP = N,7,7-Trimethyl-2,3-dioxo-N-phenylbicyclo[2.2.1.]heptan-1-carboxamid

### Example 1: Synthesis of the initiator system (CQMAP)

The acyl chloride 7,7-Dimethyl-2,3-dioxobicyclo[2.2.1]heptane-1-carbonyl chloride was dissolved in dichloromethane under a N₂ inert gas atmosphere and was mixed with pyridine at room temperature. The mixture was stirred for 1 min at room temperature before *N*-methylaniline was added. The reaction mixture was stirred for 48 h at room temperature. Then, the reaction mixture was diluted with additional dichloromethane. The resulting solution was washed two times with a saturated NaHCOs solution and after that with brine. The washed organic phase was dried over Na₂SO₄ and the product was precipitated from the solution with a mixture of *n-*hexane/ethyl acetate (70:30). As product, yellowish crystals were obtained with a yield of 89%.

### Example 2: Preparation of dental compositions A and B

Four mixtures comprising BisGMA and TEGDMA in a 70 to 30 wt% ratio, about 1.5 wt% initiator system, and either no inhibitor (A) or BHT (B) (about 0.25 wt%, in the former case replaced by more monomers) were prepared and stirred in the dark at 50°C until all compounds are homogeneously mixed (Table 1).

### Example 3: Preparation of dental compositions C and D with filler

To each of the two liquids A, B, (30 wt%) (Table 1) was added 70 wt% silanized glass filler and the mixtures were mixed (Hauschild SpeedMixer) until homogeneous pastes were received resulting in the two pastes C (from the liquid A) and D (from the liquid B).

### Example 4: Measurement of flexural strength and E-modulus

The material is filled into a metal mold of approximately 2x2x30 mm which is subsequently covered with foil and manually pressed. The material is photopolymerized for 2 min on each side using a LicuLite light oven with UV-filter. After removal from the mold, excess of material is removed by wet sanding with grit paper 1000. The specimens are conditioned in water at 37°C before they are subjected to a three-point-bending test at a Zwick universal testing machine. The span width is 20 mm, the feeding speed is 1 mm/min. For the determination of the E-modulus, the linear elastic region is used.

### Example 5: Measurement of b-value and opacity

The material is filled into a round mold of 30 mm diameter and 1 mm thickness between two sheets of foil and manually pressed. It is photopolymerized for 2 min on each side using a LicuLite light oven with UV-filter. The color measurement is done at a Datacolor 800. For the measurement of L, a, and b-value, the plates are placed on a white tile with a drop of 1 wt% sodium dodecyl sulfate solution. The result is the average of the measurement at 5 points. The opacity value is obtained in the same fashion but on a black tile.

### Example 6: Measurement of working time

Stability against ambient light or alternatively curing or working time was determined using a Suntester CPS. A droplet of the paste was squeezed between two microscopy slides and put underneath a neutral light filter (ND, D 1,0). The light intensity was adjusted to 9000 Lux. The sample was irradiated in steps of 10 s and checked if the slides can be slided against each other until polymerization. The accumulated time was noted. The average time of multiple experiments was used as working time.

### Example 7: Measurement of depth of cure

The material is filled into a cylindrical mold of 4 mm diameter and 12 mm height. The mold is covered with foil and manually pressed. It is irradiated with a dental hand lamp (SmartLite Focus) for 10 s. After removal from the mold, unpolymerized material is gently removed from the specimen with a plastic spatula. The height of the remaining cured object is measured and divided by two to obtain the depth of cure.

### Example 8: Measurement of DSC-Integral and DSC-slope

The material is filled into aluminum trays (30 mg) and inserted in a DSC-device (DSC 204 F1 Phoenix Netzsch). The temperature is equilibrated at 25°C and the measurement chamber is purged with nitrogen gas. The sample is irradiated at 1000 mW (OmniCure 2000 with 400-500 nm color filter) for 20 seconds and the polymerization heat is monitored. To correct for photothermal effects, the heat of an empty aluminum tray is subtracted. The integral of the evolved heat over time and the maximum slope are the results.

**Table 1: Composition of dental formulations A, B.**

| Composition | A | B |
|---|---|---|
| BisGMA(weight%) | 68.95 | 68.77 |
| TEGDMA (weight%) | 29.55 | 29.47 |
| DBPA (weight%) | 1.5 | 1.5 |
| BHT (weight%) | - | 0.25 |

**Table 2: Characteristics of the dental compositions of Table 1.**

| Composition | A | B |
|---|---|---|
| E-Modulus (MPa) | 752 | 611 |
| Flexural strength (MPa) | 42 | 37 |
| b-value | 2.8 | 3.8 |
| Working time (s) | >420 | >420 |
| DSC-Integral (J/g) | -33 | -23 |
| DSC-slope (mW/mg/min) | -4.5 | -3.1 |

**Table 3: Characteristics of the dental compositions of Table 1 comprising additionally 70 weight% of silanated glass filler**

| Composition | C | D |
|---|---|---|
| E-Modulus (MPa) | 6142 | 6050 |
| Flexural strength (MPa) | 102 | 91 |
| B-value | -0.37 | 0.2 |
| Working time (s) | 170 | 230 |
| Opacity (%) | 22 | 22 |
| Depth of cure (mm) | 3.3 | 2.7 |
| DSC-Integral (J/g) | -20.5 | -17.1 |
| DSC-slope (mW/mg/min) | -3.9 | -2.8 |

Fig. 1 shows the mechanical properties of dental compositions C and D of the present invention compared to the mechanical properties of a dental composition containing just camphorquinone as an initiator.

Fig. 2 shows the ¹H-NMR spectrum of N,7,7-Trimethyl-2,3-dioxo-N-phenylbicyclo[2.2.1.]heptan-1-carboxamide (CQMAP) in deuterated chloroform.

## Claims

1. A polymerizable composition comprising a mixture of
(a) at least one polymerizable monomer having a polymerizable carbon-carbon double bond;
(b) an initiator system comprising a compound of the following formula (I), or a salt thereof:
CQ-Ar (I)
wherein CQ is a group of the following formula (II) 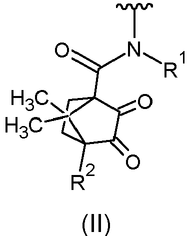
and Ar is a group of the following formula (III): 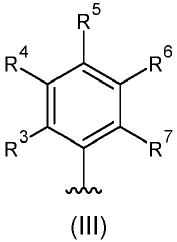
wherein
R¹ represents a hydrogen atom, or a C₁₋₆ alkyl group;
R² represents a hydrogen atom or a group CONR⁸-Ar', wherein R⁸ is a hydrogen atom, or a C₁₋₆ alkyl group, and Ar' is independently a group of formula (III);
R³, R⁴, R⁵, R⁶, and R⁷ which may be the same or different, are independently selected from a hydrogen atom, a C₁₋₈ alkyl group, a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, and a group of formula (II), wherein R¹ independently is as defined for formula (II) and R² is a hydrogen atom; with the proviso that not more than two of R³, R⁴, R⁵, R⁶, and R⁷ may be a group of formula (II) and/or a group COOR⁹.

2. The polymerizable composition according to claim 1, wherein R¹ represents a C₁₋₆ alkyl group.

3. The polymerizable composition according to claim 1 or 2, wherein R² is a hydrogen atom.

4. The polymerizable composition according to any one of the preceding claims, wherein R³, R⁴, R⁵, R⁶, and R⁷ which may be the same or different, are independently selected from a hydrogen atom and a C₁₋₈ alkyl group.

5. The polymerizable composition according to claim 4, wherein R³, R⁴, R⁵, R⁶, and R⁷ represent hydrogen atoms.

6. The polymerizable composition according to any one of claims 1 to 3, wherein one of R³, R⁴, R⁵, R⁶, and R⁷ is a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, or a group of formula (II) wherein R¹ independently is as defined for formula (II) and R² is a hydrogen atom, and the remaining of R³, R⁴, R⁵, R⁶, and R⁷ are hydrogen atoms.

7. The polymerizable composition according to any one of the preceding claims, wherein R¹ is a methyl group and R² is a hydrogen atom.

8. The polymerizable composition according to any one of the preceding claims, further comprising a polymerization inhibitor.

9. The polymerizable composition according to any one of the preceding claims, further comprising a particulate filler.

10. The polymerizable composition according to any one of the preceding claims, further comprising a polymerization accelerator, preferably a diphenyliodonium salt.

11. The polymerizable composition according to any one of the preceding claims, further comprising a solvent.

12. The polymerizable composition according to any one of the preceding claims, wherein the concentration of the compound of formula (I) is from 0.05 to 5.00 percent by weight based on the total weight of the polymerizable dental composition.

13. The polymerizable composition according to any one of the preceding claims, wherein the polymerizable dental composition is selected from a dental composite, a temporary sealing material, a dental cement, a dental adhesive, and a dental fissure sealant.

14. A compound of the following formula (I), or a salt thereof:
CQ-Ar (I)
wherein CQ and Ar are as defined in claim 1.

15. A use of the compound of formula (I) according to claim 14, or a salt thereof in a photocurable dental composition, preferably, wherein the polymerizable dental composition is selected from a dental composite, a temporary sealing material, a dental cement, a dental adhesive, and a dental fissure sealant.

## Amended claims

### Amended claims in accordance with Rule 137(2) EPC.

1. A polymerizable composition comprising a mixture of
(a) at least one polymerizable monomer having a polymerizable carbon-carbon double bond;
(b) an initiator system comprising a compound of the following formula (I), or a salt thereof:
CQ-Ar (I)
wherein CQ is a group of the following formula (II) 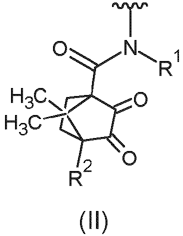 and Ar is a group of the following formula (III): 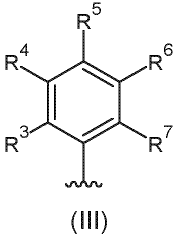 wherein
R¹ represents a hydrogen atom, or a C₁₋₆ alkyl group;
R² represents a hydrogen atom or a group CONR⁸-Ar', wherein R⁸ is a hydrogen atom, or a C₁₋₆ alkyl group, and Ar' is independently a group of formula (III);
R³, R⁴, R⁵, R⁶, and R⁷ which may be the same or different, are independently selected from a hydrogen atom, a C₁₋₈ alkyl group, a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, and a group of formula (II), wherein R¹ independently is as defined for formula (II) and R² is a hydrogen atom; with the proviso that not more than two of R³, R⁴, R⁵, R⁶, and R⁷ may be a group of formula (II) and/or a group COOR⁹.

2. The polymerizable composition according to claim 1, wherein R¹ represents a C₁₋₆alkyl group.

3. The polymerizable composition according to claim 1 or 2, wherein R² is a hydrogen atom.

4. The polymerizable composition according to any one of the preceding claims, wherein R³, R⁴, R⁵, R⁶, and R⁷ which may be the same or different, are independently selected from a hydrogen atom and a C₁₋₈ alkyl group.

5. The polymerizable composition according to claim 4, wherein R³, R⁴, R⁵, R⁶, and R⁷ represent hydrogen atoms.

6. The polymerizable composition according to any one of claims 1 to 3, wherein one of R³, R⁴, R⁵, R⁶, and R⁷ is a nitro group, a cyano group, an amino group optionally substituted by a one or two C₁₋₆ alkyl groups which may be the same or different, a group COOR⁹, wherein R⁹ is a hydrogen atom, or a C₁₋₆ alkyl group, or a group of formula (II) wherein R¹ independently is as defined for formula (II) and R² is a hydrogen atom, and the remaining of R³, R⁴, R⁵, R⁶, and R⁷ are hydrogen atoms.

7. The polymerizable composition according to any one of the preceding claims, wherein R¹ is a methyl group and R² is a hydrogen atom.

8. The polymerizable composition according to any one of the preceding claims, further comprising a polymerization inhibitor.

9. The polymerizable composition according to any one of the preceding claims, further comprising a particulate filler.

10. The polymerizable composition according to any one of the preceding claims, further comprising a polymerization accelerator, preferably a diphenyliodonium salt.

11. The polymerizable composition according to any one of the preceding claims, further comprising a solvent.

12. The polymerizable composition according to any one of the preceding claims, wherein the concentration of the compound of formula (I) is from 0.05 to 5.00 percent by weight based on the total weight of the polymerizable dental composition.

13. The polymerizable composition according to any one of the preceding claims, wherein the polymerizable dental composition is selected from a dental composite, a temporary sealing material, a dental cement, a dental adhesive, and a dental fissure sealant.

14. A use of the compound of formula (I)
CQ-Ar (I)
wherein CQ and Ar are as defined in claim 1, or a salt thereof in a photocurable dental composition, preferably a polymerizable dental composition, wherein the polymerizable dental composition is selected from a dental composite, a temporary sealing material, a dental cement, a dental adhesive, and a dental fissure sealant.
